# EUROPÄISCHE PATENTANMELDUNG

(11) **EP 0 677 532 A1**
(43) Veröffentlichungstag der Anmeldung: **18.10.1995**
(21) Anmeldenummer: 95103371.1
(22) Anmeldetag: 09.03.1995
(51) Int. Cl.: C07K 14/545, C07K 14/47, C07K 16/24, A61K 39/395

(54) **Verwendung von Il-1-Antagonisten als Arzneimittel zur Behandlung von Erkrankungen mit einem erhöhten Interleukin-6 Serumspiegel**

(30) Priorität: 16.03.1994 DE 4408890
(71) Anmelder: KNOLL AKTIENGESELLSCHAFT, D-67061 Ludwigshafen (DE)
(72) Erfinder: Stenzel, Roswitha, Dr., D-68623 Lampertheim (DE); Daum, Lothar, Dr., D-67166 Otterstadt (DE); Eiselstein, Jürgen, D-67273 Weisenheim (DE); Kaul, Martin, Dr., D-67433 Neustadt (DE); Kempeni, Joachim, Dr., D-67433 Neustadt (DE)
(74) Vertreter: Goldscheid, Bettina

(57) **Zusammenfassung**

Die Erfindung betrifft die Verwendung von Il-1-Antagonisten zur Herstellung von Arzneimitteln zur Behandlung von Erkrankungen, die durch erhöhte Interleukin-6 Serumspiegel gekennzeichnet sind.

## Beschreibung

Die vorliegende Erfindung betrifft die Verwendung von Il-1-Antagonisten bei der Behandlung von Erkrankungen mit einem erhöhten Interleukin-6 Serumspiegel.

Der Begriff Interleukin 1 (Il-1) steht für zwei Cytokine (Il-1α und Il-1β), die vor allem von Monozyten gebildet werden. Il-1-Antagonisten oder Il-1-Inhibitoren, was hier synonym verwendet werden soll, sind Substanzen, die die biologischen Aktivitäten von Il-1 spezifisch inhibieren.

Erkrankungen, die durch erhöhte Interleukin-6 Serumspiegel bei Patienten gekennzeichnet sind, sind beispielsweise Folgeerscheinungen nach Transplantationen, Autoimmunerkrankungen, Allergien, Schocklunge, Blutgerinnungsstörungen, entzündliche Knochen- und Gelenkerkrankungen und insbesondere bestimmte Formen der Sepsis.

In medizinischen Lehrbüchern wird Sepsis als ein klinischer Sammelbegriff für Zustände definiert, bei denen - ausgehend von einem Herd - bakterielle Erreger in die Blutbahn gelangen, wodurch ein großes Spektrum subjektiver und objektiver Krankheitserscheinungen ausgelöst wird. Weiterhin wird festgestellt, daß in Abhängigkeit vom Erregertyp, der Reaktionslage des Organismus, dem Primärherd und den wechselnden Organbeteiligungen das Krankheitsbild sehr variieren kann (Sturm et al. "Grundbegriffe der Inneren Medizin", 13. Auflage, Seite 570, Gustav Fischer Verlag, Stuttgart, 1984).

Für den komplexen pathophysiologischen Ablauf einer Sepsis wird die Beteiligung einer Reihe von Cytokinen diskutiert. Besonders Interleukin 1 (Il-1) und Tumor Nekrose Faktor (TNF) wird eine wichtige Rolle beim septischen Schock aufgrund tierexperimenteller Daten zugeschrieben (Beutler et al., Science 229, 869-871, 1985).

Dies hat letztlich dazu geführt, daß klinische Studien zur Behandlung von Sepsispatienten mit Il-1-Antagonisten durchgeführt wurden (Fisher et al., 13 th International Symposium on Intensive Care and Emergency Medicine, Brüssel, 23.03.93)

Die Rolle, die das Cytokin Interleukin-6 (Il-6) bei der Sepsis spielt, ist unklar und widersprüchlich. Bei einigen Sepsispatienten wurden erhöhte Serumspiegel an Il-6 gefunden (Hack et al., Blood 74, Nr.5, 1704-1710, 1989).

Waage beschreibt, daß die Konzentrationen der Cytokine Il-6 und Il-8 mit der Schwere des Schocks korrelieren; daß sie aber nicht, weder allein noch in Kombination mit TNF, die Entwicklung eines Schocksyndroms hinsichtlich Lethalität beeinflussen (Waage in "Tumor Necrosis Factors",ed. B.Beutler, Raven Press, New York,1992, Seite 275-283).

Von einigen Wissenschaftlern wird Il-6 eine günstige Rolle beim septischen Schock zugeschrieben, da Il-6 in Form einer negativen Feedback-Kontrolle die LPS-induzierte TNF Produktion hemmt (Libert et al. in "Tumor Necrosis Factor: Molecular and Cellular Biology and Clinical Relevance", ed. W. Fiers, Karger, Basel, 1993, Seite 126-131).

Überraschenderweise wurde nun gefunden, daß sich Il-1-Antagonisten besonders erfolgreich als Arzneimittel zur Behandlung von Erkrankungen einsetzen lassen, die durch erhöhte Interleukin-6 Serumspiegel gekennzeichnet sind.

Die Behandlung von Sepsis mit Il-1-Antagonisten ist gemäß dieser Erfindung besonders erfolgreich ,beispielsweise gemessen an einer deutlichen Reduzierung der Sterblichkeit, wenn solche Sepsispatienten behandelt werden, die bei Beginn der Behandlung Il-6 Werte von 500 pg/ml und mehr aufweisen. Besonders profitieren Patienten von der erfindungsgemäßen Behandlung, die Il-6 Serumwerte von größer 1000 pg/ml besitzen.

Unter erhöhten Il-6 Serumspiegeln sind solche Werte zu verstehen, die gegenüber physiologischen Serumspiegeln bei gesunden Probanden mindestens zehnfach erhöht sind.

Es wurden Serumkonzentrationen von Il-6 bei Sepsispatienten beobachtet,die bis zu 20000 fach über den Werten von gesunden Probanden lagen.

Die "normalen" Il-6 Serumwerte liegen üblicherweise unterhalb der Nachweisgrenze, was je nach verwendetem Testsystem leicht variieren kann. Sie liegen jedoch höchstens bei 20 pg/ml.

Die Serumkonzentrationen an Il-6 lassen sich mit üblichen Nachweisverfahren wie RIA oder ELISA bestimmen. Ein gut geeignetes Nachweissystem ist beispielsweise das "IL-6-EASIA" der Fa. Medgenix.

Die Konzentration an Il-6 kann auch über einen Aktivitätstest, bei dem beispielsweise C-reaktives Protein getestet wird, bestimmt werden.

Als IL-1-Antagonisten sind anti-IL-1-Antikörper, IL-1-Rezeptoren, IL-1-Bindeproteine oder solche IL-1-Derivate geeignet, die noch IL-1-Rezeptorbindung besitzen, aber keine IL-1-Aktivität mehr aufweisen. Solche Il-1-Antagonisten sind als Il-1-Rezeptorantagonisten (Il-1ra) bekannt (Eisenberg et al., Nature 343, 341, 1990; Hannum et al., Nature 343, 336, 1990.

Besonders bevorzugt für die erfindungsgemäße Verwendung sind Il-1-Rezeptorantagonisten (Il-1ra).

Die zur erfindungsgemäßen Verwendung geeigneten anti-IL-Antagonisten sind bekannt.

Zur vorliegenden Erfindung gehören pharmazeutische Zubereitungen, die neben nicht-toxischen, inerten pharmazeutisch geeigneten Trägerstoffen die IL-1-Antagonisten enthalten, sowie Verfahren zur Herstellung dieser Zubereitungen.

Die Formulierung der IL-1-Antagonisten geschieht in für biotechnisch hergestellte Wirkstoffe üblicher Weise, in der Regel als Flüssigformulierung oder Lyophilisat (siehe z.B. Hagers Handbuch der pharmazeutischen Praxis, Bd. 2, 5. Auflage, 1991, S. 720, ISBN 3-540-52459-2). Die Herstellung der oben aufgeführten pharmazeutischen Zubereitungen erfolgt in üblicher Weise nach bekannten Methoden, z.B. durch Mischen des oder der Wirkstoffe mit dem oder mit den Trägerstoffen.

Im allgemeinen hat es sich als vorteilhaft erwiesen, den oder die für die erfindungsgemäße Verwendung geeigneten Wirkstoffe in Gesamtmengen von etwa 0,1 bis etwa 1000, vorzugsweise 0,1 bis 10 mg/kg Körpergewicht je 24 Stunden, ggf. in Form mehrerer Einzelgaben oder als Dauerinfusion und ggf. über eine Therapiedauer von mehreren Tagen hinweg zur Erzielung der gewünschten Ergebnisse zu verabreichen. Die Applikation kann als intravenöse Kurzinfusion der Einzelgaben oder als kontinuierliche Langzeitinfusion der Tagesdosis über 24 Stunden erfolgen. Eine Einzelgabe enthält den oder die Wirkstoffe vorzugsweise in Mengen von etwa 0,1 bis etwa 10 mg/kg Körpergewicht. Es kann jedoch erforderlich sein, von den genannten Dosierungen abzuweichen und zwar in Abhängigkeit von Alter und Größe des zu behandelnden Patienten sowie der Art und der Schwere der zugrundeliegenden Erkrankung, der Art der Zubereitung und der Applikation des Arzneimittels sowie dem Zeitraum bzw. Intervall, innerhalb welchem die Verabreichung erfolgt.

## Patentansprüche

1. Verwendung von Il-1-Antagonisten zur Herstellung von Arzneimitteln zur Behandlung von Erkrankungen, die durch erhöhte Interleukin-6 Serumspiegel gekennzeichnet sind.

2. Verwendung nach Anspruch 1, dadurch gekennzeichnet, daß die Erkrankung eine Sepsis ist.
